# EUROPEAN PATENT APPLICATION

(11) **EP 2 311 457 A1**
(43) Date of publication of application: **20.04.2011**
(21) Application number: 09013158.2
(22) Date of filing: 19.10.2009
(51) Int. Cl.: A61K 31/4709, A61K 31/501, A61K 31/502, A61K 31/506, A61K 31/444, A61K 31/517, A61P 31/06, A61P 31/08, A61P 31/04

(54) **Pharmaceutical compositions for treating infections with drug resistant mycobacteria**

(71) Applicant: Forschungszentrum Borstel, 23845 Borstel (DE); Christian-Albrechts-Universität zu Kiel, 24118 Kiel (DE)
(72) Inventor: Seydel, Joachim, DE - 23845 Borstel (DE); Schaper, Klaus-Jürgen, DE - 23845 Borstel (DE); Clement, Bernd, DE - 24108 Kiel (DE); Rüsch-Gerdes, Sabine, DE - 21465 Reinbek (DE); Reiling, Norbert, DE - 22926 Ahrensburg (DE); Ehlers, Stefan Prof. Dr., DE - 23845 Borstel (DE)
(74) Representative: Kröncke, Rolf

(57) **Abstract**

In a first aspect, the present invention relates to pharmaceutical compositions for the treatment of infections with mycobacteria wherein said mycobacteria are resistant to at least one of the following drugs: rifampicin, isoniazid, pyrazinamide, or ethambutol, in particular, said mycobacteria are *Mycobacterium tuberculosis* (*Mtb*) complex strains. That is, the present invention relates to pharmaceutical compositions comprising as an active ingredient hydrazone compounds that have high activity against drug resistant mycobacteria, and in particular, *Mtb* complex strains resistant to isoniazid and rifampicin (multidrug-resistant *Mtb,* MDR-*Mtb*), or MDR-*Mtb* strains further being resistant to one of the injectable aminoglycosides amikacin, kanamycin or capreomycin, and to fluoroquinolones (extremely drug-resistant XDR-*Mtb*). In a further aspect, the present invention relates to the use of said hydrazone compounds for the treatment of mycobacterial infections. Moreover, the present invention relates to a method for the treatment of mycobacterial infections, in particular, MDR or XDR tuberculosis comprising the step of administering to an individual in need thereof an effective amount of a hydrazone compound of general formula I.

## Description

In a first aspect, the present invention relates to pharmaceutical compositions for the treatment of infections with mycobacteria wherein said mycobacteria are resistant to at least one of the following drugs: rifampicin, isoniazid, pyrazinamide, or ethambutol, in particular, said mycobacteria are *Mycobacterium tuberculosis (Mtb)* complex strains. That is, the present invention relates to pharmaceutical compositions comprising as an active ingredient hydrazone compounds that have high activity against drug resistant mycobacteria, and in particular, *Mtb* complex strains resistant to isoniazid and rifampicin (multidrug-resistant *Mtb,* MDR-*Mtb),* or MDR*-Mtb* strains further being resistant to one of the injectable aminoglycosides amikacin, kanamycin or capreomycin, and to fluoroquinolones (extremely drug-resistant XDR*-Mtb).* In a further aspect, the present invention relates to the use of said hydrazone compounds for the treatment of mycobacterial infections. Moreover, the present invention relates to a method for the treatment of mycobacterial infections, in particular, MDR or XDR tuberculosis comprising the step of administering to an individual in need thereof an effective amount of a hydrazone compound of general formula I.

### Prior art

Mycobacterial infections, such as tuberculosis or leprosy, have been known for a long time. Infections with mycobacteria still represent a worldwide threat to human health. Although the cause of tuberculosis and leprosy has been known since 1892 and 1873, respectively, treatment of mycobacterial infections still represents a major challenge in medicine. Tuberculosis (TB), is an airborne, infectious bacterial disease caused by *Mycobacterium tuberculosis (Mtb),* which most commonly affects the lungs. Tuberculosis can also be caused by other mycobacterial species such as *M*. *bovis, M. africanum, M. canetti, M. microti* and *M. pinepedii,* which all belong to the so-called *M. tuberculosis* complex. Bacteria of the *Mtb* complex are transmitted from person to person via droplets from the throat and lungs of people with the active respiratory disease. Someone in the world is newly infected with *Mtb* every second. Overall, one third of the world's population is currently infected with *Mtb.* Approximately ten percent of individuals infected with *Mtb* will develop disease. The symptoms of active TB of the lung are coughing, sometimes with sputum or blood, chest pains, weakness, weight loss, fever and night sweats. Tuberculosis is usually treatable with a six-month course of a combination of antibiotics. Left untreated, each person with active TB disease will infect on average between 10 and 15 people every year. The mortality of TB, if left untreated, is in the order of 20-30 % and can be much higher, if the patient suffers from an immunodeficiency, such as HIV infection.

It is estimated that 1.7 million deaths resulted from TB cases in 2007. Both, the highest number of deaths and the highest mortality per capita are in the African region. The TB epidemic in Africa grew rapidly during the 1990ies. It is estimated that in the next 20 years over one billion people will be newly infected with *Mtb,* and 35 million people will die from the disease.

Until 60 years ago, no drugs were available to cure TB. Today, drugs such as isoniazid, rifampicin, rifabutin, pyrazinamide, ethambutol, streptomycin, fluoroquinolones, para-aminosalicylic acid, ethionamide, protionamide, cycloserine, amikacin, capreomycin, kanamycin etc. are available for treating TB. Among these, isoniazid, rifampicin, ethambutol, and pyrazinamide are the first-line drugs of choice, which are administered in a combination regimen of three or more of the aforesaid drugs, either as a single drug formulation or as a fixed dose combination of two or more of the aforesaid drugs.

Now, strains that are resistant to a single drug have been documented in every country surveyed. Strains resistant to the two most important first line drugs, isoniazid and rifampicin (termed multidrug-resistant or MDR-*Mtb*), have emerged in the last two decades. More recently, strains of *Mtb* resistant not only to isoniazid and rifampicin, but also to fluoroquinolones and one of the injectable aminoglycosides such as kanamycin, amikacin or capreomycin, have emerged (termed extremely drug-resistant or XDR-*Mtb*), and there are reports of *Mtb* strains that are resistant to all major anti-TB drugs.

For drug-sensitive *Mtb*, therapy has to be performed for six months: two months of a four-drug regimen consisting of isoniazid, rifampicin, ethambutol and pyrazinamide, followed by four months of isoniazid and rifampicin. Some of these drugs have unpleasant side effects so that the long course of treatment leads to non-compliance of patients. Due to the high number of *Mtb* in the infected organs, individuals with *Mtb* always harbour strains with naturally occurring mutations that are resistant to one or two drugs. When non-compliant patients do not take the required combination of at least three drugs outlined above, selection of *Mtb* resistant strains occurs. This has happened in many countries where follow-up of treated patients was difficult (due to collapsing public health services, e.g. prisons in the former Soviet Union) or where drug supplies were limited. These *Mtb* strains, resistant to one or more drugs, are now spreading throughout the world.

That is, drug resistant TB is caused by inconsistent or partial treatment when patients do not take the prescribed drugs regularly for the required period because they start to feel better, because doctors and health workers prescribed the wrong treatment regimens, or because the drugs supply is unreliable. In particular, more severe forms of drug-resistant Tb include MDR-TB. Rates of MDR-TB are high in some countries, especially in the countries of the former Soviet Union, and threaten TB control efforts. In particular, TB and especially MDR-TB moves to Europe and America from Eastern Europe and Asia, thus, spreading MDR-TB.

Drug resistant TB requires extensive chemotherapy (sometimes up to two years of treatment) with the second-line anti-TB drugs which are more costly than first-line drugs and which produce more severe adverse drug reactions than the first-line drugs. When second-line drugs are also misused, MDR-TB can develop into XDR-TB.

Because XDR-TB is resistant to the first and the second-line drugs, treatment options are seriously limited. It is therefore vital that TB control is managed properly and that drugs are available which are also applicable for MDR-TB and XDR-TB. Hence, there is an ongoing need for new drugs able to treat drug resistant mycobacteria, in particular of the *Mtb* complex.

Almost 20 years ago, new substituted 2-acylpyridine-α-(N)-hetarylhydrazones and drug formulations containing the same being useful for the treatment of microbial infections and, in particular, mycobacterial diseases but also for the treatment of malaria, were disclosed in EP 313630, and in US Patents 4,997,835 and 5,084,458. Therein substituted 2-acylpyridine-α-(N)-hetarylhydrazones are described which represent inhibitors of mycobacterial ribonucleotide reductase (RDR). Already in 1986, Schaper, Seydel et al. (Leprosy Rev., 57, Suppl. 3, 254-264), described the development of inhibitors of *M. leprae* ribonucleotide reductase including hetarylhydrazone derivatives. It was also demonstrated that these compounds show anti-mycobacterial activity against drug-sensitive mycobacteria, including *Mtb* strain H37Rv. However, EP 313630, US Patents 4,997,835 and 5,084,458 are focused on the successful treatment of leprosy in an animal model.

### Brief description of the present invention

It has now been found that the said hetarylhydrazones are powerful inhibitors of *Mtb* strains of the MDR- and XDR-type. In addition it was found that the ranking in activity in minimum inhibitory concentrations (MIC) values against the *M*. *leprae* model strain *M*. *lufu* differs from that found for hetarylhydrazones against MDR-TB strains. Therefore, compounds showing highest activity against MDR-TB were selected and optimized. In addition, new hetarylhydrazones not described in EP 313630 and US patents 4,997,835 and 5,084,458 are described herein.

In a first aspect, the present invention relates to drugs and products able to treat infections with mycobacteria wherein said mycobacteria are resistant against first-line anti-TB antibiotics, such as rifampicin or isoniazid, the two most important anti-TB drugs.

That is, in a first aspect, the present invention relates to a pharmaceutical composition for the treatment of infections with mycobacteria wherein said mycobacteria are resistant to at least one of rifampicin, isoniazid, pyrazinamide, or ethambutol as first-line anti-TB antibiotics, preferably, rifampicin or isoniazid, comprising as an active ingredient a compound of general formula I 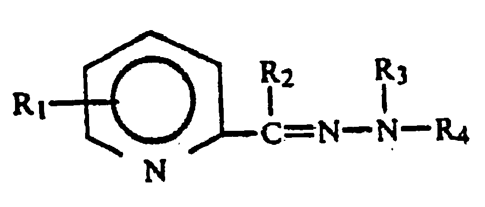 wherein R₁ is hydrogen, halogen, and an alkyl group with 1 to 8 C-atoms, a benzyloxy-, an amino-, or acetamino group;
R₂ is hydrogen, an alkyl group with 1 to 8 C-atoms, a benzyl or a phenyl group; R₃ is hydrogen;
R₄ is selected from any one of general formula II, III, IV 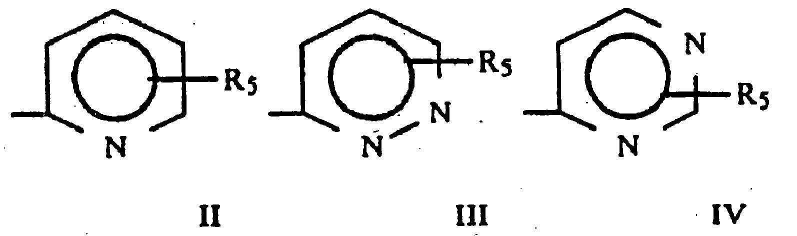 wherein R₅ is hydrogen, halogen, C₁-C₄ alkyl group, C₁-C₄ alkoxy group, benzo group, substituted benzo group substituted with C₁-C₃ alkyl, dialkylamino-group or substituted dialkylamino group substituted with C₁-C₃ alkyl; or salts or solvates thereof.

The pharmaceutical composition is particularly useful for the treatment of MDR tuberculosis and XDR tuberculosis. The active ingredient according to the present invention may be combined with at least a second anti-mycobacterial agent known in the art, for example, anti-mycobacterial agents acting as inhibitors of the folate synthase and/or the dihydrofolic acid reductase and/or of the DNA synthesis and/or RNA synthesis.

In a further aspect, the present invention relates to the use of a compound of general formula I as defined above for the treatment of MDR tuberculosis, in particular, XDR tuberculosis.

Moreover, the present invention relates to a method for the treatment of mycobacterial infections whereby said mycobacteria are resistant to at least rifampicin or isoniazid, comprising the step of administering to an individual in need thereof an effective amount of a compound of general formula I as defined above or salts or solvates thereof.

### Brief description of the drawings

**Figure 1:** Figure 1 shows the growth inhibitory activity of derivative PH22-33 on *Mtb*-infected macrophages. Murine bone marrow derived macrophages were infected with *Mtb* H37Rv in the absence or presence of increasing concentrations of PH22-33. On day 7 p.i. cells were lysed and colony forming units (CFU) were determined. No toxicity was observed at the concentrations indicated as measured by MTT assay.
**Figure 2:** Figure 2 provides the structures of compounds particularly useful according to the present invention.

### Detailed description of the present invention

In a first aspect, the present invention relates to a pharmaceutical composition for the treatment of infections with mycobacteria wherein said mycobacteria are resistant to at least one of the first-line anti-TB antibiotics, i.e. rifampicin, isoniazid, ethambutol, or pyrazinamide comprising as an active ingredient a compound of general formula I 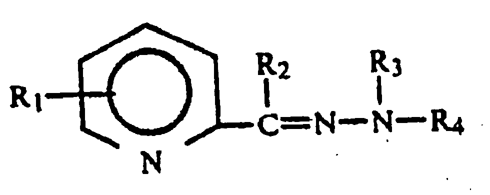 wherein R₁ is hydrogen, halogen, or an alkyl group with 1 to 8 C-atoms, a benzyloxy-, an amino-, or acetamino group;
R₂ is hydrogen, an alkyl group with 1 to 8 C-atoms, a benzyl or a phenyl group; R₃ is hydrogen;
R₄ is selected from any one of general formula II, III, IV 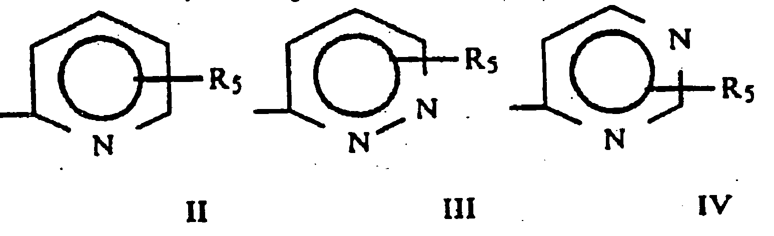 wherein R₅ is hydrogen, halogen, C₁-C₄ alkyl group, C₁-C₄ alkoxy group, benzo group, substituted benzo group, substituted with C₁-C₃ alkyl, dialkylamino-group or substituted dialkylamino group, substituted with C₁-C₃ alkyl; or salts or solvates thereof.

It is within the scope of the present invention that when chiral atoms are present in the hydrazones according to the present invention, the hydrazones may be present as racemates or in form of the stereoisomeric R or S form, respectively. Examples of nonchiral stereoisomers are PH22-27&-37; - 29&-30; -45&-46; -50&-51; -6&-52, respectively.

The present inventors recognised that, surprisingly, compounds of general formula I display a very low MIC (minimum inhibitory concentration) against MDR-*Mtb* and XDR-*Mtb* clinical isolates.

In this connection, the terms "MDR tuberculosis" and "XDR tuberculosis" are used herein as defined by the WHO. Namely, MDR tuberculosis relates to the form of tuberculosis where *Mtb* is resistant to at least isoniazid and rifampicin, and XDR tuberculosis to *Mtb* resistant against isoniazid, rifampicin plus at least one of the injectable aminoglycosides and a fluoroquinolone.

The term "polyresistant" refers to mycobacterial strains that are resistant to at least two of the first line drugs except for the combined resistance against rifampicin and isoniazid which is referred to as MDR.

The term "resistant to least one of a first-line anti-TB antibiotic" refers to a resistance to at least one of rifampicin, isoniazid, pyrazinamide, or ethambutol.

The term "MIC" refers to the "minimum inhibitory concentration" of a compound. That is, the minimum inhibitory concentration is the lowest concentration of a drug that leads to a full stop of bacterial multiplication.

The term "C₁-C₈ alkyl" comprises C₁-C₆ alkyl, for example C₁-C₄ alkyl groups. C₁-C₄ alkyl is methyl, ethyl, propyl, namely n-propyl or iso-propyl, or butyl, e.g. butyl, iso-butyl or tert-butyl. C₁-C₃ alkyl is methyl, ethyl, n-propyl or iso-propyl.

The term "halogen" includes fluoro, chloro, bromo, iodo, trifluoromethyl or trifluoromethoxy substituents.

Surprisingly, the present inventors recognised that hydrazone compounds which are described as useful active ingredients for the treatment of microbial diseases in general, are effective in inhibiting growth of resistant to at least one of a first-line anti-TB antibiotic, MDR and XDR forms of *Mtb* complex, and are therefore useful for the treatment of mycobacterial infections with polyresistant mycobacteria, in particular, of the MDR and XDR type.

This is particularly noteworthy since many other well-known anti-mycobacterially active drugs fail to inhibit the growth of XDR-*Mtb*. There are few salvage therapies, for example with linezolid or para-amino-salicylic acid, which both incur a high risk of side effects. Not to bound to theory, it is assumed that the low MIC values of hydrazones against MDR-*Mtb* isolates as described herein circumvent these side effects, indicating the usefulness of these compounds as novel powerful drugs to treat MDR-TB and XDR-TB.

In a preferred embodiment, the pharmaceutical composition according to the present invention is useful for the treatment of infections with polyresistant and MDR strains of the *Mtb* complex. In particular, the present invention provides a pharmaceutical composition useful for the treatment of XDR tuberculosis.

In a preferred embodiment, the active ingredient of general formula I present in the pharmaceutical composition according to the present invention is defined as follows: R₁ is hydrogen, R₂ is phenyl, alkyl, benzyl, R₄ is a residue of general formula II with R₅ being a benzo group, or, alternatively, R₁ is a C₂-alkyl group, R₂ is hydrogen, R₄ is a residue of general formula IV with R₅ being methyloxy.

Other preferred compounds are as follows: pyridine-2-aldehyde-1'-phthalazinylhydrazone (PH 22-1), 2-acetylpyridine-1'-phthalazinylhydrazone (PH 22-3), 2-acetylpyridine-2'-quinolylhydrazone (PH 22-4), 5-ethylpyridine-2-aldehyde-1'-phthalazinylhydrazone (PH 22-5), 5-benzyloxypyridine-2-aldehyde-1'-phthalazinylhydrazone (PH 22-6), 4-methyl-pyridine-2-aldehyde-1'-phthalazinylhydrazone (PH 22-8), 2-benzoylpyridine-2'-pyridylhydrazone (PH 22-9), 5-ethyl-pyridine-2-aldehyde-2'-pyridylhydrazone (PH 22-13), 5-methylpyridine-2-aldehyde-2'-quinolylhydrazone (PH 22-14), 2-benzoylpyridine-2'-quinolylhydrazone (PH 22-15), 5-methyl-pyridine-2-aldehyde-1'-phthalazinylhydrazone (PH 22-16), 4-acetylaminopyridine-2-aldehyde-1'-phthalazinylhydrazone (PH 22-18), 4-acetylaminopyridine-2-aldehyde-2'-quinolylhydrazone (PH 22-19), 4-acetylamino-2-acetylpyridine-1'-phthalazinylhydrazone (PH 22-20), 2-benzoylpyridine-1'-phthalazinylhydrazone (PH 22-22), 4-aminopyridine-2-aldehyde-1'-phthalazinylhydrazone (PH 22-23), 2-acetyl-4-aminopyridine-1'-phthalazinylhydrazone (PH 22-24), ethyl-2-pyridylketone-1'-phthalazinylhydrazone (PH 22-25), pyridine-2-aldehyde-6'-chloro-4'-pyrimidylhydrazone (PH 22-26), pyridine-2-aldehyde-1'-isoquinolylhydrazone (PH 22-27), pyridine-2-aldehyde-6'-chloro-2'-pyridylhydrazone (PH 22-29), pyridine-2-aldehyde-5'-chloro-2'-pyridylhydrazone (PH 22-30), pyridine-2-aldehyde-6'-methyl-2'-pyridylhydrazone (PH 22-31), 2-acetylpyridine-6'-chloro-4'-pyrimidylhydrazone (PH 22-32), 5-ethylpyridine-2-aldehyde-6'-methoxy-4'-pyrimidylhydrazone (PH 22-33 ), 2-acetylpyridine-4'-quinazolylhydrazone (PH 22-34), isoquinoline-1-aldehyde-2'-pyridylhydrazone (PH 22-37), di-2-pyridyl-ketone-6'-chloro-4'-pyrimidylhydrazone (PH 22-39), di-2-pyridyl-ketone-5'-chloro-2'-pyridylhydrazone (PH 22-40), 2-benzoylpyridine-6'-chloro-3'-pyridazinylhydrazone (PH 22-45), 2-benzoylpyridine-6'-chloro-4'-pyrimidylhydrazone (PH 22-46), 2-acetylpyridine-6'-chloro-3'-pyridazinylhydrazone (PH 22-47), 5-benzyloxypyridine-2-aldehyde-2'-pyridylhydrazone (PH 22-49), 5-benzyloxypyridine-2-aldehyde-6'-chloro-4'-pyrimidylhydrazone (PH 22-50), 5-benzyloxypyridine-2-aldehyde-6'-chloro-3'-pyridazinylhydrazone (PH 22-51), 5-benzyloxypyridine-2-aldehyde-4'-quinazolinylhydrazone (PH 22-52), ethyl-2-pyridylketone-6'-chloro-4'-pyrimidylhydrazone (PH 22-53), propyl-2-pyridylketone-6'-chloro-4'-pyrimidylhydrazone (PH 22-54), benzyl-2-pyridylketone-6'-chloro-4'-pyrimidylhydrazone (PH 22-55).

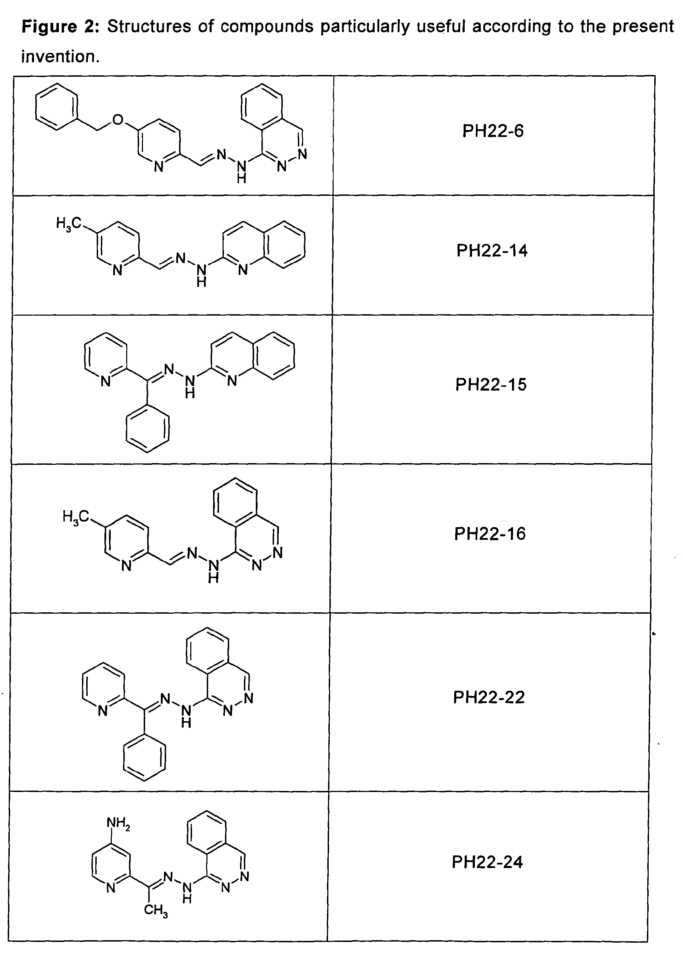

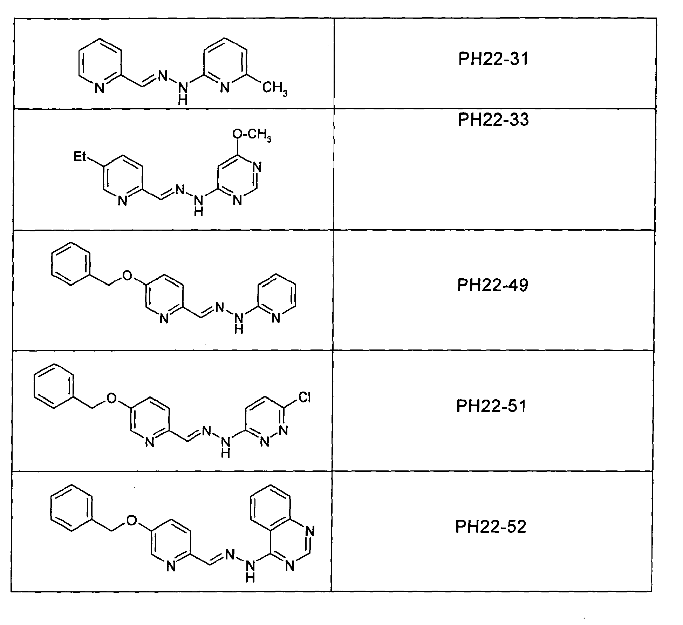

The particularly preferred compounds according to the present invention are shown in Figure 2.

In a particularly preferred embodiment, the pharmaceutical composition contains as active ingredient 5-ethylpyridine-2-aldehyde-6'-methoxy-4'-pyrimidylhydrazone (PH 22-33) or 2-benzoylpyridine-2'-quinolylhydrazone (PH 22-15).

Thus, the present invention relates to pharmaceutical compositions comprising compounds according to formula (I) or salts or solvates thereof, and, optionally, a pharmaceutically acceptable carrier. Such pharmaceutical compositions comprise a therapeutically effective amount of the compounds and, optionally, a pharmaceutically acceptable carrier. The pharmaceutical composition may be administered to a patient with a physiologically acceptable carrier, as described herein.

In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds according to formula (I), or salts or solvates thereof, preferably in purified form, together with a suitable amount of carrier so as to provide the form for proper administration to the patient. The formulation should suit the mode of administration.

Typically, a pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lidocaine to ease pain at the site of the injection. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The term "administered" means administration to an individual of a therapeutically effective dose of the aforementioned pharmaceutical composition comprising the compound according to formula (I), or salts and solvates thereof as defined herein. "Therapeutically or pharmaceutically effective amount" as applied to the compositions of the instant invention refers to the amount of composition sufficient to induce a desired biological result. That result can be alleviation of the signs, symptoms, or causes of a disease, or any other desired alteration of a biological system. That is, by "therapeutically effective amount" is meant a dose that produces the effects for which it is administered. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

In vitro assays may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The compounds of general formula I may be in the form of salts or solvates (e.g. hydrates).

The pharmaceutical compositions and the methods are applicable to both human therapy and veterinary applications. The compounds described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt%. The agents may be administered alone or in combination with other treatments.

The administration of the pharmaceutical composition can be done in a variety of ways as discussed above, including, but not limited to, orally, subcutaneously, intravenously, intra-arterial, intranodal, intramedullary, intrathecal, intraventricular, intranasally, conjunctival, intrabronchial, transdermally, intrarectally, intraperitoneally, intramuscularly, intrapulmonary, vaginally, rectally, or intraocularly. In some instances, the pharmaceutically effective agent may be directly applied as a solution dry spray.

The present inventors found that compounds according to the present invention display a very low MIC in a range of about 0.125 µg/ml to 1.0 µg/ml not only in drug-sensitive *Mtb*, but also in MDR-*Mtb* strains. The low MIC value demonstrates a high efficiency in inhibiting the RDR enzyme and, thus, demonstrates its usefulness in therapy of tuberculosis of resistant *Mtb*.

Moreover, it is possible to combine the compounds according to the present invention with known anti-mycobacterial agents, e.g. one or more inhibitors of the folate synthase and/or of the dihydrofolic acid reductase and/or of the DNA synthesis and/or RNA synthesis as it is known from EP313630 or US 5,084,458, respectively. The pharmaceutical composition comprising two different active ingredients may be adapted to allow simultaneous, sequential or separate administration of the two active ingredients.

In another aspect, the present invention relates to the use of the compound of general formula I as defined herein for the treatment of resistant tuberculosis, in particular, MDR tuberculosis and, preferably, XDR tuberculosis.

In addition, the present invention relates to a method for the treatment of infections with mycobacteria whereby said mycobacteria are resistant against at least one first-line anti-Tb antibiotic, like rifampicin or isoniazid, comprising the step of administering to a person in need thereof an effective amount of a compound of general formula I as defined herein or a salt or solvate thereof.

Preferably, the method relates to the treatment of MDR tuberculosis and, in particular, XDR tuberculosis.
Preferably, the administration is effected by the oral route.

The preferred dose quantity, composition and preparation of pharmaceutical formulations which are to be used in each case as described above depends upon the patient, its age, weight and individual condition, the individual pharmacokinetic data and the mode of administration. The skilled artisan will be in a position to determine the effective amount of the drug based on the above parameters.

The following examples serve to illustrate the invention without limiting the invention's scope.

### Examples

### Example 1

### Drug susceptibility

Drug susceptibility testing (DST) was performed with the broth-based radiometric BACTEC 460TB system (Becton Dickinson Diagnostic Systems, Sparks, MD) which is considered the laboratory "gold standard". The standard protocol for DST and determination of MICs was strictly followed as recommended by the manufacturers. The results are shown in Table 1 below: Radiometric determination of minimal inhibitory concentrations of ten PH22 derivatives. *Mtb* 09457/06 represents an XDR strain while *Mtb* H37Rv ATCC 272945 is a representative of a drug-sensitive strain with no known resistance.

| **Table 1 Radiometric determination of minimal inhibitory concentrations [µg/ml] of ten PH22 derivatives using the BACTEC460 and the MGIT960 system.** | | | | | | | | | | |
|---|---|---|---|---|---|---|---|---|---|---|
| **Com pound/ Strain** | **PH22 -33** | **PH22 -14** | **PH22 -15** | **PH22 -16** | **PH22 -31** | **PH22 -49** | **PH22 -51** | **PH22 -52** | **PH22 -6** | **PH22 -22** |
| ***Mtb* 09457/06 XDR*** | <0.125 | 0.5 | 0.25 | < 1.0 | 1.0 | 1.0 | 1.0 | 1.0 | 1.0 | 5.0 |
| ***Mtb* H37Rv ATCC 272945** | <0.125 | 0.5 | 0.25 | <1.0 | <1.0 | 5.0 | 1.0 | 1-5.0 | 5.0 | 5.0 |

| | | | | | | | | | | |
|---|---|---|---|---|---|---|---|---|---|---|
| * resistant to: Isoniazid, rifampicin, rifabutin, streptomycin, ethambutol, pyrazinamide, ofloxacin, capreomycin, protionamide | | | | | | | | | | |

### Example 2

### Growth inhibitory activity on Mtb infected macrophages

**Mice and generation of macrophages.** C57Bl/6 mice were maintained under specific pathogen-free conditions. Mice were sacrificed 8 to 16 weeks after birth. For the generation of bone derived macrophages marrow cells were extracted by rinsing of femora and tibia. Cells were centrifuged for 10 min at 200 x g and resuspended in Dulbecco's modified Eagle medium (DMEM) containing 10% (v/v) heat-inactivated fetal calf serum (FCS), 100 U/ml of penicillin G, 100 µg/ml of streptomycin, 4 mM L-glutamine, 10 mM HEPES, 1 mM sodium pyruvate and 50 ng/ml of recombinant human macrophage colony-stimulating factor. Cells were first cultured on a Nunclon^{™} Δ surface dish (Nunc, Roskilde, Denmark). After 24 h of cultivation non-adherent cells were harvested. Cells were transferred into fresh medium to a culture dish for 6 days at a maximum density of 1x10⁶ cells per ml medium. Fresh medium was added every 3 days. Cell viability was determined by trypan blue staining.

**Bacteria and in vitro infection.** *Mtb* strain (H37Rv, ATCC 27294, Manassas, VA) was grown in Middlebrook 7H9 medium containing 10% OADC (oleic acid, albumin, dextrose, catalase), 0.5% glycerol, and 0.05% Tween 80. Midlog phase cultures were harvested, and aliquots of defined CFU were frozen at -80°C until use. For *in vitro* experiments, bacterial aliquots were thawed, centrifuged for 10 min at 835 x g and resuspended in PBS.

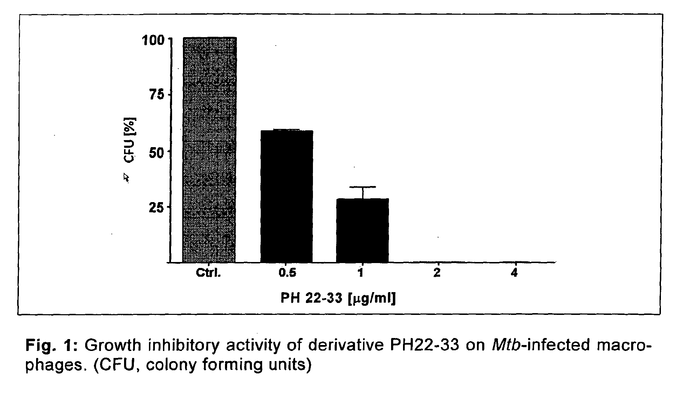

In vitro infection. 1 x 10⁶ murine macrophages were seeded in 0.5 mL macrophage culture medium in 24-well flat-bottom Nunclon^{™} Surface plates, cultured (37°C, 5% CO₂ overnight and infected for 4 h with *Mtb* H37Rv at a multiplicity of infection of 1:1. Cells were subsequently washed 3 times with Hanks' balanced salt solution (HBSS) at 37°C to remove non-phagocytosed bacteria. Subsequently cells were incubated in the absence or presence of PH22-33 at the indicated concentrations for 7 days. After 3 days 500µl fresh medium was added. On day 7 p.i. macrophages cultures were lysed by addition of 0.02% saponin at 37°C for 15 min. Lysates were serially diluted in 0.05% Tween 80, and plated twice on 7H10 agar. After 3 weeks at 37°C the CFUs were counted. Depicted in Figure 1 is the CFU in [%]. 100% represents the bacterial growth in the absence of antibiotics.

## Claims

1. A pharmaceutical composition for the treatment of mycobacterial infections whereby said mycobacteria are resistant to at least one first-line anti-TB drug, comprising as an active ingredient a compound of general formula (I): 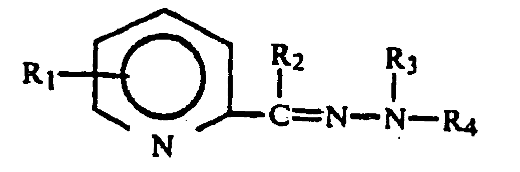 wherein R₁ is hydrogen, halogen, and an alkyl group with 1 to 8 C-atoms, a benzyloxy-, an amino-, or acetamino group;
R₂ is hydrogen, an alkyl group with 1 to 8 C-atoms, a phenyl or a benzyl group;
R₃ is hydrogen;
R₄ is selected from any one of general formula II, III, IV 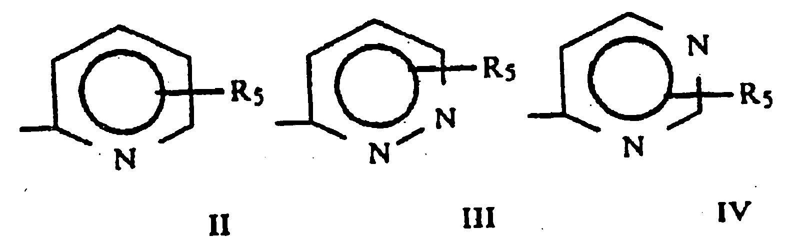 wherein R₅ is hydrogen, halogen, C₁-C₄ alkyl group, C₁-C₄ alkoxy group, benzo group, substituted benzo group substituted with C₁-C₃ alkyl, dialkylamino group or substituted dialkylamino group substituted with C₁-C₃ alkyl; or salts or solvates thereof.

2. The pharmaceutical composition according to claim 1 for the treatment of MDR tuberculosis.

3. The pharmaceutical composition according to any one of claim 1 or 2 for the treatment of XDR tuberculosis.

4. The pharmaceutical composition according to any one of the preceding claims wherein R₁ is hydrogen, R₂ is alkyl, phenyl or benzyl, R₄ is the residue of general formula II with R₅ being a benzo group, or R₁ is a C₂ alkyl group, R₂ is hydrogen, R₄ is a residue of general formula IV with R₅ being methyloxy.

5. The pharmaceutical composition according to any one of the preceding claims adapted for oral administration.

6. The pharmaceutical composition according to any one of the preceding claims containing at least one further anti-mycobacterial agent, e.g. one or more inhibitors of the folate synthase and/or of the dihydrofolic acid reductase and/or of the DNA-synthesis and/or RNA synthesis.

7. The pharmaceutical composition according to any one of the preceding claims wherein at least one further anti-mycobacterial agent and more specifically at least one of the following compounds is present: 1st line oral antituberculosis agents Isoniazid, rifampicin, ethambutol, pyrazinamide; injectable antituberculosis agents, in particular, streptomycin, amikacin, capreomycin, kanamycin; fluoroquinolones, in particular,ciprofloxacin, ofloxacin, moxifloxacin, oral 2nd line antituberculosis agents, in particular, rifabutin, protionamide, ethionamide, cycloserine, PAS, thioacetazone and other ant-ituberculosis agents, in particular, linezolid, clofazimine, amoxicillin/clavulanate, or a derivative of diaminodiphenylsulphone.

8. The pharmaceutical composition according to claim 6 or 7, wherein the compound of general formula I and at least one further compound are adapted for simultaneous, sequential or separate administration.

9. Use of a compound of general formula I as defined in any one of the preceding claims for the treatment of MDR tuberculosis, in particular, XDR tuberculosis.

10. A method for the treatment of infections with mycobacteria whereby said mycobacteria are resistant against at least rifampicin or isoniazid comprising the step of administering to an individual in need thereof an effective amount of a compound of general formula I as defined in any one of claims 1 to 4 or a salt or solvate thereof.

11. The method according to claim 10 whereby the administration is effected by the oral route.
